(19) Europäisches Patentamt
European Patent Office
Office européen des brevets

(11) Numéro de publication: **0 275 759 B1**

(12) **FASCICULE DE BREVET EUROPEEN**

(45) Date de publication de fascicule du brevet: **29.01.92**

(51) Int. Cl.5: **C07D 211/56**, C07D 223/12, A61K 31/445, A61K 31/55

(21) Numéro de dépôt: **87402885.5**

(22) Date de dépôt: **17.12.87**

(54) **3-pipéridineamines ou 3-azépineamines substituées, leur préparation et leurs applications en thérapeutique.**

(30) Priorité: **23.12.86 FR 8618083**

(43) Date de publication de la demande: **27.07.88 Bulletin 88/30**

(45) Mention de la délivrance du brevet: **29.01.92 Bulletin 92/05**

(84) Etats contractants désignés: **AT BE CH DE ES FR GB GR IT LI NL SE**

(56) Documents cités: **US-A- 4 097 481**

(73) Titulaire: **RIOM LABORATOIRES- C.E.R.M. "R.L.-CERM" - (S.A.)
Route de Marsat B.P. 140
F-63203 Riom Cédex(FR)**

(72) Inventeur: **Carlier, Patrick
La Gravière 10, Fonfreyde
F-63140 Chatel-Guyon(FR)**
Inventeur: **Simond, Jacques Aimé Louis
Rue des Thuilets, Mirefleurs
F-63730 Les-Martres-De-Veyre(FR)**
Inventeur: **Monteil, André Jean-Claude
Route de Prompsat, Les Grosliers
F-63140 Chatel-Guyon(FR)**

(74) Mandataire: **Hermans, Franciscus G.M. et al
Patent Department AKZO N.V. Pharma Division P.O. Box 20
NL-5340 BH Oss(NL)**

EP 0 275 759 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouvelles 3-piperidineamines ou 3-azephineamines substituées, leur préparation et leurs applications en thérapeutique.

Plus précisément, les 1-alkyl-2-alkoxyméthyl-N-phényl N-benzyl ou N-benzoyl-3-pipéridineamines ou 3-azépineamines substituées selon l'invention répondent à la formule générale suivante :

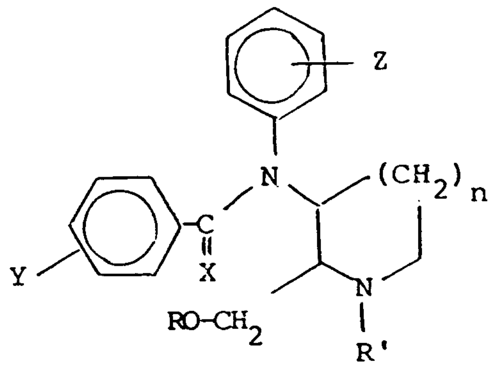

dans laquelle R et R′ représentent chacun un radical alkyle ou cycloalkyle ayant 1 à 7 atomes de carbone ; X représente - O - ou $H_2$ ; Y et Z représentent l'hydrogène ou un ou plusieurs radicaux choisis parmi les radicaux halogéno, hydroxy, alkyl linéaire ou ramifié ayant 1 à 6 atomes de carbone, alkoxy ayant 1 à 6 atomes de carbone, trifluromethyle ou méthylènedioxy, n pouvant prendre les valeurs 2 ou 3.

L'invention concerne aussi les sels desdits composés avec les acides organiques ou inorganiques pharmaceutiquement acceptables, tels que les acides chlorhydrique, fumarique, maléique, citrique ou succinique, ces acides n'étant mentionnés qu'à titre illustratif mais sans constituer une limitation.

Selon une réalisation préférée, le substituant R représente l'un des radicaux isobutyle, méthyle ou cyclohexyle ; R′ représente le radical méthyle ou n-propyle.

Les composés de l'invention comportant des atomes de carbone asymétriques, le racémique et/ou les isomères optiques séparés ainsi qu'un mélange de ceux-ci font également partie de l'invention.

Les études pharmacologiques ont montré que les composés de l'invention possédaient d'intéressantes propriétés permettant leur application en thérapeutique humaine dans le traitement des troubles cardiovasculaires.

Les composés de l'invention peuvent être préparés à partir de 2-(α-hydroxy-β-alkoxy) éthyl pyrrolidine ou-pipéridine, selon le schéma réactionnel ci-après :

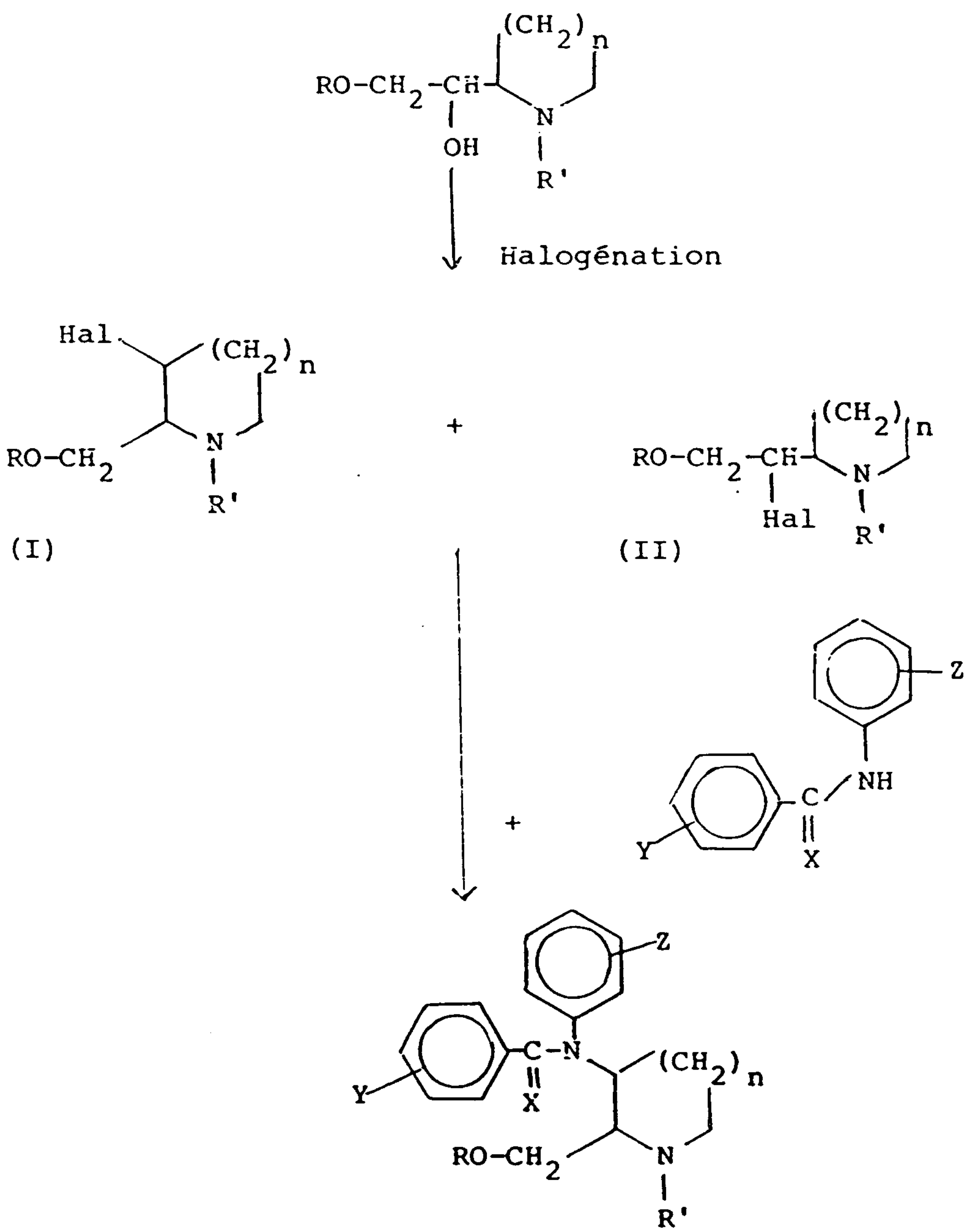


(I) (II)

Dans une première étape, on effectue l'halogénation d'une 2-(α-hydroxy-β-alkoxy) éthyl pyrrolidine ou pipéridine au moyen d'un agent habituel d'halogénation tel que $SOCl_2$, $PBr_3$ dans un solvant tel que le chloroforme, à une température comprise entre la température ambiante et la température de reflux de solvant.

Le dérivé halogéné formé peut avoir la structure I ou la structure II, ou bien être un mélange des deux types de structure.

Dans la première étape, on obtient de façon prépondérante le composé de type I (correspondant à un agrandissement de cycle azoté) lorsque n = 2, alors qu'on obtient seulement des composés de type II lorsque n = 3, l'agrandissement du cycle s'effectuant dans la deuxième étape.

Dans la deuxième étape, le produit obtenu à l'étape précédente est condensé avec une benzylaniline ou benzoylaniline substituée de préférence en présence d'une base forte telle que l'amidure de sodium ou de lithium. Selon une variante du procédé, cette deuxième étape peut être réalisée par transfert de phase en présence d'une base forte telle qu'une solution d'hydroxyde de sodium à 50-70 % et d'un catalyseur tel que le chlorure de benzyl triéthylammonium, en ajoutant, si la viscosité du milieu l'exige, un solvant tel que le toluène ou le chlorure de méthylène. Selon une autre variante du procédé, lorsque X représente $H_2$, les composés peuvent encore être obtenus par réduction des homologues correspondants (X représente = O).

Cette réduction peut être effectuée au moyen des agents habituels tels que l'hydrure de lithium-aluminium, le diborane, dans un solvant tel que l'éther ou le tétrahydrofuranne. A l'issue de cette étape, les composés de l'invention sont extraits du mélange réactionnel par les méthodes habituelles (par exemple extraction à l'éther ou au chlorure de méthylène) puis purifiés par chromatographie liquide préparative. Ils peuvent également être extraits par formation et cristallisation d'un sel pharmaceutiquement acceptable.

Les exemples ci-après illustrent plus en détails la préparation des composés de l'invention.

EXEMPLE 1 : 1-méthyl-2-[(2-méthylpropoxy) méthyl]-N-benzyl N-(3,4-méthylènedioxy) phényl-3-pipéridineamine

Dans une première étape, on a introduit 100 g (0,5 M) de 1-méthyl-2-(α-hydroxy-β-isobutoxy) éthyl pyrrolidine dans un réacteur contenant 1 l de chloroforme anhydre, puis chauffé à 50° C et introduit goutte à goutte une solution de 82 ml de chlorure de thionyle dans 80 ml de chloroforme anhydre, et maintenue 4 heures en chauffant à reflux de solvant.

On a ensuite évaporé le solvant et repris le résidu par 1 l d'acide chlorhydrique à 3 %, puis lavé au chlorure de méthylène, alcalinisé à la lessive d'hydroxyde de sodium puis extrait au chlorure de méthylène. Après séchage sur sulfate de sodium et évaporation du solvant, on a distillé le résidu et obtenu 64 g de 1-méthyl 2-isobutoxyméthyl-3-chloro pipéridine ayant pour point d'ébullition $Eb_{0,5}$ = 92-93° C.

Dans une deuxième étape, à une suspension de 2,1 g (0,09 M) d'amidure de lithium dans 80 ml de toluène, on a ajouté à froid 8,35 g (0,045 M) de 3,4-méthylènedioxy-benzylaniline et 9,5 g (0,045 M) du dérivé chloré préparé à la première étape en solution dans 20 ml de toluène. On a ensuite laissé la réaction se poursuivre en chauffant à reflux pendant 5 heures, puis laissé le milieu réactionnel refroidir, et ensuite hydrolysé par 20 ml d'une solution aqueuse saturée de chlorure d'ammonium. Après filtration, lavage à l'eau, séchage sur sulfate de sodium et évaporation du solvant, on a distillé le résidu. On a ensuite purifié par chromatographie liquide préparative sur gel de silice en utilisant comme solvant le mélange ci-après : Hexane : 85 %, Ethanol : 7,5 %, Isopropanol : 7,5 %.

On a ainsi obtenu 2 g de composé du titre ayant pour point d'ébullition $Eb_{0,8}$ = 230° C, pour indice de réfraction $n_D^{20}$ = 1,5600 et pour analyse élémentaire

| | C % | H % | N % |
|---|---|---|---|
| Théorie | 73,13 | 8,34 | 6,82 |
| Trouvé | 73,43 | 8,46 | 6,89 |

Ce composé a également été obtenu, selon les méthodes usuelles sous forme de chlorhydrate ayant pour point de fusion F = 148° C.

EXEMPLE 2 : 1-méthyl-2-[(2-méthylpropoxy) méthyl]-N-benzoyl N-phényl-3-pipéridineamine

Dans une première étape, on a préparé la 1-méthyl-2-isobutoxyméthyl-3-chloro pipéridine comme indiqué dans l'exemple 1.

Dans la deuxième étape, on a introduit dans un réacteur 20 g d'hydroxyde de sodium, 20 ml d'eau, 2 g de chlorure de benzyltriéthylammonium, 20,6 g de benzanilide et 50 ml de toluène, puis on a ajouté goutte à goutte une solution de 20 g de dérivé halogéné, préparé dans la première étape, dans 50 ml de toluène.

On a ensuite chauffé le mélange à 85° C pendant 3 heures, puis laissé refroidir, décanté et lavé la phase aqueuse au chlorure de méthylène. Les phases organiques ont été rassemblées, lavées à l'eau, séchées sur sulfate de sodium, puis le solvant a été évaporé.

On a ainsi obtenu 34,9 g d'un résidu brut qu'on a agité 1 heure avec 500 ml d'une solution d'acide chlorhydrique à 10 %, puis neutralisé à l'hydroxyde de sodium, extrait au chlorure de méthylène et séché. Après évaporation du solvant, on a ajouté 600 ml d'hydroxyde de sodium à 5 % et chauffé à 80-90° C pendant 10 heures. Après refroidissement, on a extrait au chlorure de méthylène, séché sur sulfate de sodium et évaporé le solvant.

On a ainsi obtenu 26,5 g de produit qu'on a distillé, puis purifié par chromatographie liquide préparative sur gel de silice en utilisant comme solvant la composition ci-après :
Hexane : 84,9 %, Ethanol : 7,5 %, Isopropanol : 7,5 %, Ammoniaque : 0,1 %.

On a ainsi obtenu 3 g de composé du titre ayant pour point d'ébullition $Eb_{0,7}$ = 203-206° C, pour point de fusion F = 80° C et pour analyse élémentaire :

| | C % | H % | N % |
|---|---|---|---|
| Théorie<br>Trouvé | 75,75<br>76,11 | 8,47<br>8,57 | 7,36<br>7,35 |

EXEMPLE 3 : 1-propyl-2-méthoxyméthyl-N-benzoyl-N-phényl-3-azépineamine

Dans une première étape, on a introduit dans un réacteur contenant 320 ml de chlororome anhydre, 32 g (0,16 M) de 1-propyl-2-($\alpha$-hydroxy-$\beta$-méthoxy) éthyl pipéridine, porté à 50° C puis ajouté goutte à goutte 32 ml de chlorure de thionyle dans 32 ml de chloroforme, puis chauffé à reflux pendant 10 h 30. Après séparation et distillation, on a obtenu 24,25 g de 1-propyl-2-($\alpha$-chloro-$\beta$-méthoxy)-éthyl pipéridine ayant pour point d'ébullition $Eb_{0,5}$ = 95° C.

Dans une deuxième étape, en procédant comme indiqué dans l'exemple 2, on a mis à réagir le dérivé précédemment obtenu avec la benzanilide par réaction de transfert de phase. En partant de 19,75 g (0,09 M) de dérivé chloré et de 20,6 g (0,1 M) de benzanilide, après avoir chauffé 18 heures à 80-90° C, puis isolé et purifié le composé formé par les moyens indiqués, on a obtenu 15 g de composé du titre ayant pour point d'ébullition $Eb_{0,04}$ = 175-180° C.

EXEMPLE 4 : 1-propyl-2-méthoxyméthyl-N-benzyl-N-phényl-3-azepineamine

Dans un réacteur contenant une suspension de 4,95 g de borohydrure de sodium dans 100 ml de tétrahydrofuranne, on a ajouté, sous atmosphère d'azote, 15 g du composé préparé à l'exemple 3 en solution dans 35 ml de tétrahydrofuranne, en maintenant la température à 0° C. On a ensuite ajouté goutte à goutte 29,6 ml de borotrifluorure éthérate dans 39 ml de tétrahydrofuranne. Après avoir laissé le milieu réactionnel revenir à la température ambiante, on a ajouté 69 ml d'acide chlorhydrique à 10 %. Le solvant est ensuite éliminé par distillation à pression atmosphérique, le résidu refroidi puis alcalinisé à la lessive d'hydroxyde de sodium puis extrait au chlorure de méthylène. Après séchage et évaporation du solvant, le produit est distillé, puis purifié par chromatographie liquide préparative sur gel de silice en utilisant comme solvant le méthanol à 2 % puis 4 % dans le dichlorométhane.

On obtient ainsi 5 g de composé du titre ayant pour point d'ébullition $Eb_{0.1}$ = 172-174° C et pour analyse élémentaire

| | C % | H % | N % |
|---|---|---|---|
| Théorie<br>Trouvé | 78,64<br>77,83 | 9,35<br>9,43 | 7,64<br>7,58 |

De la même façon, on a préparé d'autres composés selon l'invention, dont l'identification est donnée dans le tableau I ci-après, et les caractéristiques physicochimiques dans le tableau I bis.

TABLEAU I

| COMPOSE N° | R | R' | X | Y | Z | n |
|---|---|---|---|---|---|---|
| 1 | 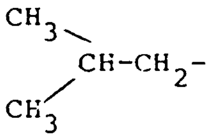 | $-CH_3$ | $H_2$ | H | H | 2 |
| 2 | 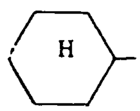 | $-CH_3$ | O | H | H | 2 |
| 3 (Exemple 2) | 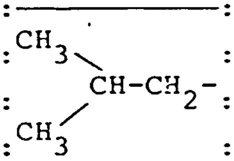 | $-CH_3$ | O | H | H | 2 |
| 4 (Exemple 1) | 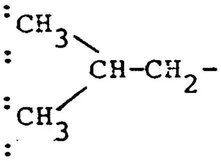 | $-CH_3$ | $H_2$ | H | $-O-CH_2-O-$ 3,4 | 2 |
| 5 | 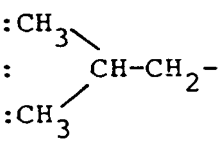 | $-CH_3$ | $H_2$ | H | H | 3 |
| 6 | 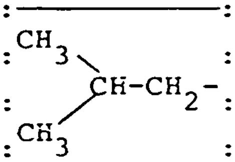 | $-CH_3$ | O | H | H | 3 |
| 7 (Exemple 3) | $CH_3-$ | $-(CH_2)_2-CH_3$ | O | H | H | 3 |
| 8 (Exemple 4) | $CH_3-$ | $-(CH_2)_2-CH_3$ | $H_2$ | H | H | 3 |
| 9 | 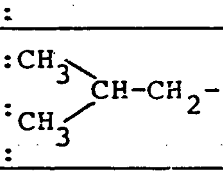 | $-CH_3$ | $H_2$ | $4-CH_3$ | $4-CH_3$ | 2 |

TABLEAU I Bis

| COMPOSE N° | $n_D^{20}$ | F°C | Eb mmHg °C |
|---|---|---|---|
| 1 | 1,5560 | / | $Eb_{0,5}$= 198-202°C |
| 2 | / | F = 98°C | $Eb_{0,5}$= 220-222°C |
| 3 (Exemple 2) | / | F = 80°C | $Eb_{0,7}$= 203-206°C |
| 4 (Exemple 1) | 1,5600 | Chlorhydrate F = 148°C | $Eb_{0,8}$= 230°C |
| 5 | 1,5620 | / | $Eb_{0,4}$= 186-192°C |
| 6 | 1,5460 | / | $Eb_{0,4}$= 185-187°C |
| 7 (Exemple 3) | / | / | $Eb_{0,04}$=175-180°C |
| 8 (Exemple 4) | / | | $Eb_{0,1}$= 172-174°C |
| 9 | 1,5480 | / | $Eb_{0,1}$= 190-195°C |

Les composés de l'invention se sont révélés posséder d'intéressantes propriétés anti-angineuses, avec des effets bradycardisants, antitachycardisants et coronarodilatateurs.

L'activité anticalcique a été recherchée selon la technique de Van Rossum (Arch. Int. Pharmacodyn. Ther. 143, 299-330, 1963). Pour évaluer l'activité anticalcique au niveau cardiaque, on a utilisé le muscle papillaire de lapin stimulé électriquement (fréquence 1,5 Hz ; 5 ms d'impulsion de 15 v). Pour l'activité au niveau vasculaire, on a utilisé l'aorte de lapin découpée en spirale et maintenue dans une solution dépourvue de $Ca^{++}$ et enrichie en $K^{+}$ (6 mg/l de KCl). Les mesures ont été effectuées 15 minutes après avoir ajouté les composés à la solution. Les paramètres classiques de pharmacologie moléculaire sont rapportés dans le tableau II.

La recherche de l'activité anti-angineuse a été évaluée en recherchant les effets hémodynamiques chez le chien anesthésié. L'animal est anesthésié au chloralose (100 $mg.kg^{-1}$ I.V.) et les paramètres suivants sont enregistrés :

. fréquence cardiaque à l'aide d'électrodes à ECG sous-cutanées reliées à un cardiotachymètre,
. le débit artériel coronaire à l'aide d'un débitmètre électromagnétique,
. la pression artérielle à l'aide d'un cathéter Mikro-tip au niveau de la crosse aortique,
. l'inotropisme évalué par rapport au temps de la pression intraventriculaire gauche enregistrée à l'aide d'un cathéter Mikro-tip dans le ventricule gauche,
. l'action anti-tachycardisante (inhibition des effets chronotropes positifs de l'isoprénaline).

Ces paramètres sont enregistrés en continu sur un dynographe BECKMAN. Les composés sont administrés par voie I.V. à la dose de 5 $mg.kg^{-1}$.

Les résultats sont exprimés en pourcentage de variation, la durée daction étant indiquée entre parenthèses, en minutes (Tableau III).

TABLEAU II

| COMPOSE N° | ACTIVITE ANTICALCIQUE | |
|---|---|---|
| | CARDIAQUE | VASCULAIRE |
| 1 | 4,1 | 5,3 |
| 4 | <4 | 6,2 |
| 5 | <4 | 4,7 |
| 8 | <4 | 4,2 |

TABLEAU III

| COMPOSE N° | ACTIVITE HEMODYNAMIQUE | | | | |
|---|---|---|---|---|---|
| | FREQUENCE CARDIAQUE | DEBIT CORONAIRE | HYPOTENSION | INOTROPISME | ANTI-TACHYCARDIE |
| 1 | - 24 (>60) | + 56 (0,5) | - 50 (10) | - 48 (>60) | - 50 (40) |
| 2 | - 21 (>40) | + 103 (2) | - 73 (20) | - 76 (>39) | - 80 (>40) |
| 3 | - 22 (>45) | + 75 (4) | - 50 (12) | - 71 (>40) | - 13 (17) |
| 4 | - 72 (>40) | + 54 (4) | - 60 (25) | - 69 (30) | - 65 (40) |
| 5 | - 11 (7) | + 27 (1) | - 36 (1) | - 24 (16) | 0 |
| 8 | - 10 (23) | - 18 (4) | 0 | - 14 (25) | 0 |

Ces résultats montrent que les composés de l'invention possèdent des propriétés anticalciques aussi bien sur le muscle cardiaque que sur le muscle vasculaire.

En ce qui concerne les effets hémodynamiques, tous ces composés peuvent être, à des degrés différents, des anti-angineux et/ou anti-ischémiques en raison de leurs activités bradycardisante, coronarodilatatrice et anti-tachycardisante.

Leur action sur la pression artérielle permet également leur application comme antihypertenseurs.

Parmi les composés de l'invention, celui qui présente le plus d'intérêt est le composé n° 4 avec un tropisme uniquement vasculaire et des activités hémodynamiques importantes.

La toxicité a été évaluée par voie orale chez la souris et on n'a observé aucun décès jusqu'à la dose de 500 $mg.kg^{-1}$.

Cet ensemble de propiétés pharmacologiques permet donc l'application des composés de l'invention en thérapeutique humaine en tant que médicament pour le traitement des troubles cardiovasculaires, tel que l'angine de poitrine, l'ischémie ou l'hypertension.

Les composés de l'invention peuvent être administrés par voie orale ou parentérale à des doses journalières comprises entre 0,5 mg et 10 mg par kg de poids corporel selon la méthode d'administration.

En thérapeutique humaine, on utilise de préférence une dose journalière comprise entre 50 et 500 mg.

Associés aux excipients pharmaceutiques habituels, les composés de l'invention, ou leurs sels, peuvent être mis sous forme unitaires tels que pilules, comprimés, comprimés enrobés, ou bien conditionnés en capsules. En utilisant des liquides appropriés, les composés de l'invention peuvent aussi être utilisés en préparation injectable, ou en préparation orale sous forme de solutions, suspensions ou émulsions.

**Revendications**

**Revendications pour les Etats contractants suivants : AT, BE, CH, DE, FR, GB, IT, LI, NL, SE,**

**1.** Composés de formule :

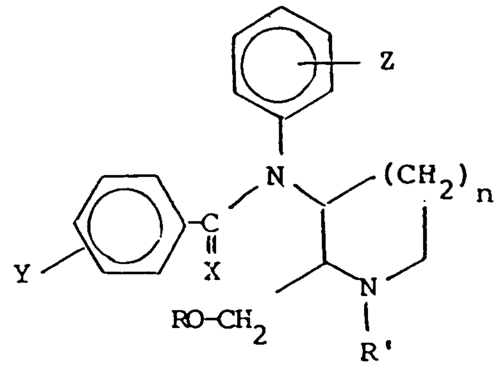

dans laquelle R et R′ représentent chacun un radical alkyle ou cycloalkyle ayant 1 à 7 atomes de carbone ; X représente - O - ou $H_2$ ; Y et Z représentent l'hydrogène ou un ou plusieurs radicaux choisis parmi les radicaux halogéno, hydroxy, alkyl linéaire ou ramifié ayant 1 à 6 atomes de carbone, alkoxy ayant 1 à 6 atomes de carbone, trifluorméthyle ou méthylènedioxy, n pouvant prendre les valeurs 2 ou 3, et leurs sels pharmaceutiquement acceptables.

**2.** Composés selon la revendication 1, caractérisés en ce que R représente un radical isobutyle, méthyle ou cyclohexyle.

**3.** Composés selon la revendication 1, caractérisés en ce que R′ représente le radical méthyle ou n-propyle.

**4.** Composé selon la revendication 1, caractérisé en ce qu'il est le 1-méthyl-2-[(2-méthyl-propoxy) méthyl]-N-benzyl-N-(3,4-méthylènedioxy) phényl-3-pipéridineamine ou un de ses sels.

**5.** Procédé pour l'obtention des composés selon l'une quelconque des revendications 1 à 4,caractérisé en ce qu'on fait agir un dérivé halogéné de formule I ou II

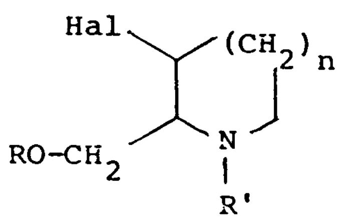

(I)

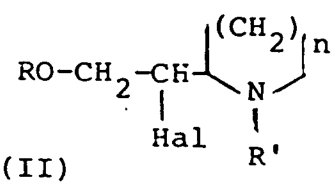

(II)

(dans lesquels R et R′ ont les significations de la revendication 1 et " Hal " désigne un halogène) avec une benzylaniline ou benzoylaniline substituée de formule

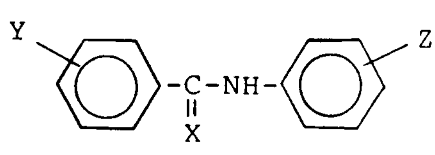

(dans laquelle X, Y et Z ont les significations de la revendication 1)
éventuellement suivi par une réduction du composé ainsi obtenu lorsque X = O, et/ou par la transformation en un sel pharmaceutiquement acceptable.

**6.** Procédé selon la revendication 5, caractérisé en ce que la réaction est effectuée en présence d'une base forte telle que l'amidure de sodium ou l'amidure de lithium.

**7.** Procédé selon la revendication 5, caractérisé en ce que la réaction est effectuée par transfert de phase en présence d'une base forte et d'un catalyseur tel que le chlorure de benzyltriéthylammonium.

**8.** Procédé selon la revendication 5, caractérisé en ce qu'on obtient les dérivés halogéno de formule I ou II en traitant une 2-(α-hydroxy-β-alkoxy) éthyl pyrroidine ou-pipéridine par un agent d'halogénation.

**9.** Procédé selon la revendication 8, caractérisé en ce que l'halogénation est effectuée au moyen du chlorure de thionyl.

**10.** Composition pharmaceutique utile en tant que médicament, notamment pour le traitement des troubles cardiovasculaires, caractérisée en ce qu'elle contient à titre de principe actif au moins un des composés selon les revendications 1 à 4.

**Revendications pour les Etats contractants suivants : ES, GR**

**1.** Procédé pour l'obtention des composés de formule:

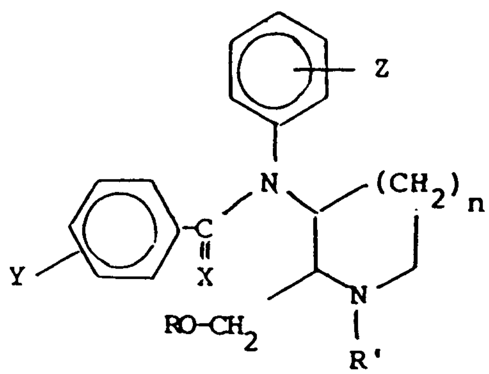

dans laquelle R et R' représent chacun un radical alkyle ou cycloalkyle ayant 1 à 7 atomes de carbone; X représente - O - ou $H_2$; Y et Z représentent l'hydrogène ou un ou plusieurs radicaux choisis parmi les radicaux halogéno, hydroxy, alkyl linéaire ou ramifié ayant 1 à 6 atomes de carbone, alkoxy ayant 1 à 6 atomes de carbone, trifluorométhyle ou méthylènedioxy, n pouvant prendre les valeurs 2 ou 3, et leurs sels pharmaceutiquement acceptables, caractérisé en ce qu'on fait agir un dérivé halogéné de formule I ou II

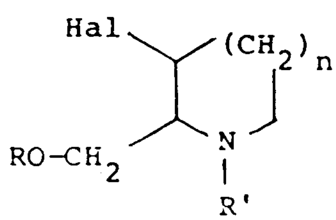

(I)

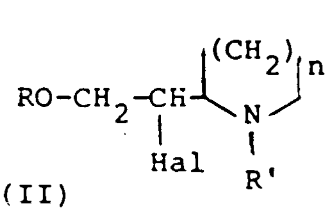

(II)

(dans lesquels R et R' ont les significations données ci-dessus et "Hal" désigne un halogène) avec une benzylaniline ou benzoylaniline substituée de formule

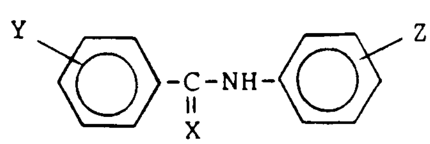

(dans laquelle X, Y et Z ont les significations données ci-dessus)
éventuellement suivi par une réduction du composé ainsi obtenu lorsque X = O, et/ou par la transformation en un sel pharmaceutiquement acceptable.

2. Procédé selon la revendication 1, caractérisés en ce que R représente un radical isobutyl, méthyle ou cyclohexyle.

3. Procédé selon la revendication 1, caractérisés en ce que R' représente le radical méthyle ou n-propyle.

4. Procédé selon la revendication 1, caractérisé en ce qu'il est le 1-méthyl-2-[(2-méthyl-propoxy)méthyl]-N-benzyl-N-(3,4-méthylènedioxy)phényl-3-pipéridine-amine ou un de ses sels.

5. Procédé selon la revendication 1, caractérisé en ce que la réaction est effectuée en présence d'une base forte telle que l'amidure de sodium ou l'amidure de lithium.

6. Procédé selon la revendication 1, caractérisé en ce que la réaction est effectuée par transfert de phase en présence d'une base forte et d'un catalyseur tel que le chlorure de benzyltriéthylammonium.

7. Procédé selon la revendication 1, caractérisé en ce qu'on obtient les dérivés halogéno de formule I ou II en traitant une 2-($\alpha$-hydroxy-$\beta$-alkoxy)éthyl pyrrolidine ou-pipéridine par un agent d'halogénation.

8. Procédé selon la revendication 7, caractérisé en ce que l'halogénation est effectuée au moyen du chlorure de thionyl.

**Claims**
**Claims for the following Contracting States : AT, BE, CH, DE, FR, GB, IT, LI, NL, SE**

1. Compounds of the formula:

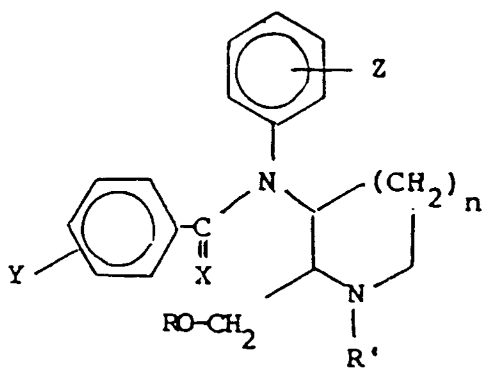

in which R and R' each represent an alkyl or cycloalkyl radical having 1 to 7 carbon atoms; X represents - O - or $H_2$; Y and Z represent hydrogen or one or more radicals chosen from halogen radicals, hydroxy radicals, linear or branched alkyl radicals having 1 to 6 carbon atoms, alkoxy radicals having 1 to 6 carbon atoms, or trifluoromethyl or methylene-dioxy radicals, it being possible for n to adopt the values 2 or 3, and their pharmaceutically acceptable salts.

2. Compounds according to Claim 1, characterised in that R represents an isobutyl, methyl or cyclohexyl radical.

3. Compounds according to Claim 1, characterised in that R' represents a methyl or n-propyl radical.

4. Compound according to Claim 1, characterised in that it is 1-methyl-2-[(2-methyl-propoxy)methyl]-N-benzyl-N-(3,4-methylenedioxy) phenyl-3-piperidineamine or one of its salts.

5. Process for the preparation of the compounds according to one of Claims 1 to 4, characterised in that a halogenated derivative of the formula I or II

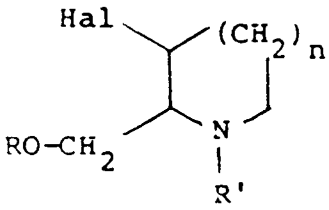

(I)

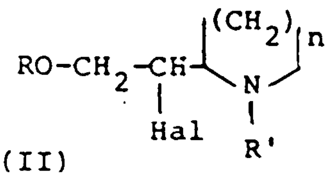

(II)

(in which R and R' have the meanings of Claim 1 and "Hal" represents a halogen) is reacted with a substituted benzylaniline or benzoylaniline of the formula

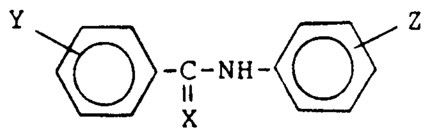

(in which X, Y and Z have the meanings of Claim 1), followed if desired by reduction of the compound thus obtained, if X = O, and/or by conversion into a pharmaceutically acceptable salt.

**6.** Process according to Claim 5, characterised in that the reaction is carried out in the presence of a strong base such as sodium amide or lithium amide.

**7.** Process according to Claim 5, characterised in that the reaction is carried out by phase transfer in the presence of a strong base and a catalyst such as benzyltriethylammonium chloride.

**8.** Process according to Claim 5, characterised in that the halogen derivatives of the formula I or II are obtained by treating a 2-($\alpha$-hydroxy-$\beta$-alkoxy)ethyl-pyrrolidine or -piperidine with a halogenating agent.

**9.** Process according to Claim 8, characterised in that halogenation is effected by means of thionyl chloride.

**10.** Pharmaceutical composition suitable as a medicament, especially for the treatment of cardiovascular disorders, characterised in that it contains at least one of the compounds according to Claims 1 to 4 as the active substance.

**Claims for the following Contracting States : ES, GR**

**1.** Process for the preparation of the compounds of the formula:

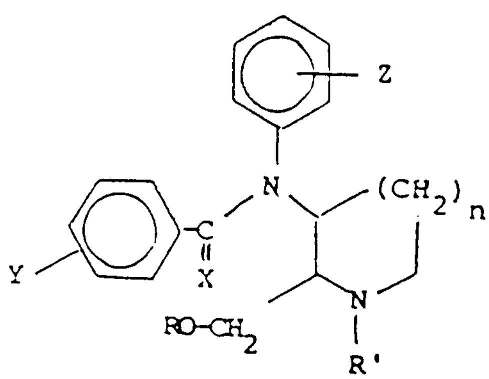

in which R and R' each represent an alkyl or cycloalkyl radical having 1 to 7 carbon atoms; X represents - O - or $H_2$; Y and Z represent hydrogen or one or more radicals chosen from halogen

radicals, hydroxy radicals, linear or branched alkyl radicals having 1 to 6 carbon atoms, alkoxy radicals having 1 to 6 carbon atoms, or trifluoromethyl or methylene-dioxy radicals, it being possible for n to adopt the values 2 or 3, and their pharmaceutically acceptable salts, characterised in that a halogenated derivative of the formula I or II

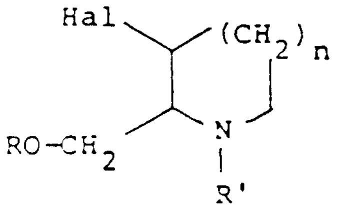

(I)

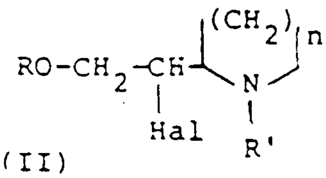

(II)

(in which R and R' have the meanings given above and "Hal" represents a halogen) is reacted with a substituted benzylaniline or benzoylaniline of the formula

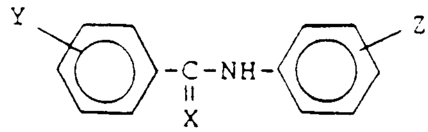

(in which X, Y and Z have the meanings given above, followed if desired by reduction of the compound thus obtained, if X = O, and/or by conversion into a pharmaceutically acceptable salt.

2. Process according to Claim 1, characterised in that R represents an isobutyl, methyl or cyclohexyl radical.

3. Process according to Claim 1, characterised in that R' represents a methyl or n-propyl radical.

4. Process according to Claim 1, characterised in that it is 1-methyl-2-[(2-methyl-propoxy)methyl]-N-benzyl-N-(3,4-methylenedioxy)phenyl-3-piperidineamine or one of its salts.

5. Process according to Claim 1, characterised in that the reaction is carried out in the presence of a strong base such as sodium amide or lithium amide.

6. Process according to Claim 1, characterised in that the reaction is carried out by phase transfer in the presence of a strong base and a catalyst such as benzyltriethylammonium chloride.

7. Process according to Claim 1, characterised in that the halogen derivatives of the formula I or II are obtained by treating a 2-(α-hydroxy-β-alkoxy)ethyl-pyrrolidine or -piperidine with a halogenating agent.

8. Process according to Claim 7, characterised in that halogenation is effected by means of thionyl chloride.

**Patentansprüche**
**Patentansprüche für folgende Vertragsstaaten : AT, BE, CH, DE, FR, GB, IT, LI, NL, SE,**

1. Verbindungen der Formel:

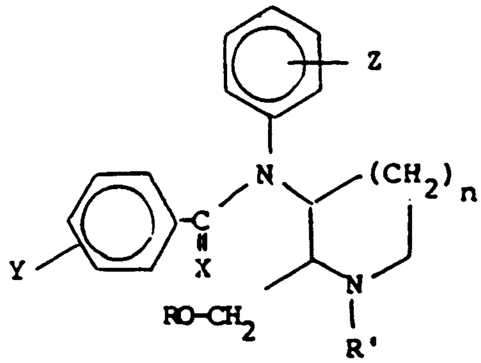

worin R und R' jeweils einen Alkyl- oder Cycloalkylrest mit 1 bis 7 Kohlenstoffatomen; X -O- oder $H_2$ ; Y und Z Wasserstoff oder einen oder mehrere unter Halogen-, Hydroxyl-, geradkettigen oder verzweigten Alkylresten mit 1 bis 6 Kohlenstoffatomen, Alkoxyresten mit 1 bis 6 Kohlenstoffatomen, Trifluormethyl- oder Methylendioxyresten ausgewählte Reste darstellen, wobei n den Wert 2 oder 3 annehmen kann, sowie ihre pharmazeutisch verträglichen Salze.

2. Verbindungen nach Anspruch 1, dadurch gekennzeichnet, daß R einen Isobutyl-, Methyl- oder Cyclohexylrest darstellt.

3. Verbindungen nach Anspruch 1, dadurch gekennzeichnet, daß R' den Methyl- oder n-Propylrest darstellt.

4. Verbindung nach Anspruch 1, dadurch gekennzeichnet, daß es sich um 1-Methyl-2-[(2-methylpropoxy)-methyl]-N-benzyl-N-(3,4-methylendioxyd)phenyl-3-piperidinamin oder ein Salz davon handelt.

5. Verfahren zur Herstellung von Verbindungen nach einem der Ansprüche 1 bis 4, dadurch gekennzeichnet, daß man ein halogeniertes Derivat der Formel I oder II

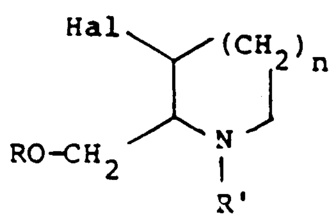

(I)

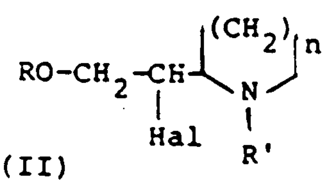

(II)

(worin R und R' die Bedeutungen des Anspruchs 1 aufweisen und "Hal" ein Halogen bezeichnet) mit einem substituierten Benzylanilin oder Benzoylanilin der Formel

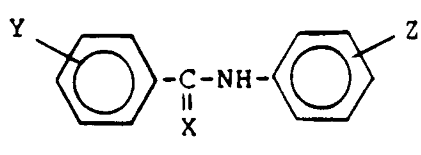

(worin X, Y und Z die Bedeutungen des Anspruchs 1 aufweisen) umsetzt, und gegebenenfalls danach eine Reduktion der so erhaltenen Verbindung, falls X = O, und/oder die Umwandlung in ein pharmazeutisch verträgliches Salz ausführt.

6. Verfahren nach Anspruch 5, dadurch gekennzeichnet, daß die Umsetzung in Anwesenheit einer starken Base wie Natriumamid oder Lithiumamid erfolgt.

7. Verfahren nach Anspruch 5, dadurch gekennzeichnet, daß die Umsetzung durch Phasentransfer in

Anwesenheit einer starken Base und eines Katalysators wie Benzyltriethylammoniumchlorid erfolgt.

**8.** Verfahren nach Anspruch 5, dadurch gekennzeichnet, daß man die Halogenderivate der Formel I oder II durch Behandlung eines 2-(α-Hydroxy-β-alkoxy)ethyl-pyrrolidins oder-piperidins mit einem Halogenierungsmittel erhält.

**9.** Verfahren nach Anspruch 8, dadurch gekennzeichnet, daß die Halogenierung mittels Thionylchlorid erfolgt.

**10.** Als Arzneimittel geeignete Zusammensetzung, insbesondere zur Behandlung von Herz-Kreislauf-Erkrankungen, dadurch gekennzeichnet, daß sie als Wirkstoff mindestens eine der Verbindungen nach Ansprüchen 1 bis 4 enthält.

**Patentansprüche für folgende Vertragsstaaten : ES, GR**

**1.** Verfahren zur Herstellung von Verbindungen der Formel:

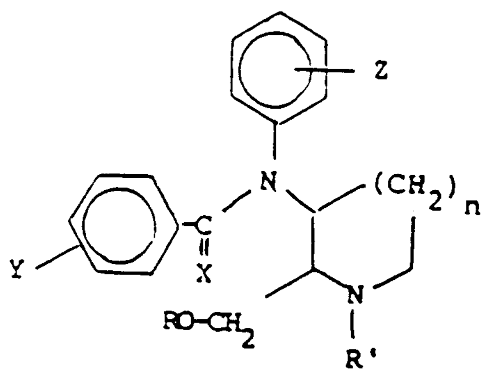

worin R und R' jeweils einen Alkyl- oder Cycloalkylrest mit 1 bis 7 Kohlenstoffatomen; X -O- oder $H_2$ ; Y und Z Wasserstoff oder einen oder mehrere unter Halogen-, Hydroxyl-, geradkettigen oder verzweigten Alkylresten mit 1 bis 6 Kohlenstoffatomen, Alkoxyresten mit 1 bis 6 Kohlenstoffatomen, Trifluormethyl- oder Methylendioxyresten ausgewählte Reste darstellen, wobei n den Wert 2 oder 3 annehmen kann, sowie ihre pharmazeutisch verträglichen Salze, dadurch gekennzeichnet, daß man ein halogeniertes Derivat der Formel I oder II

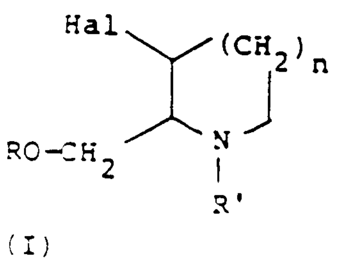

(I)

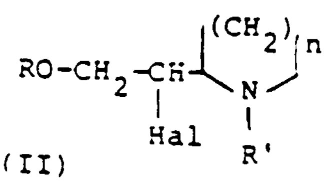

(II)

(worin R und R' die obengenannten Bedeutungen aufweisen und "Hal" ein Halogen bezeichnet) mit einem substituierten Benzylanilin oder Benzoylanilin der Formel

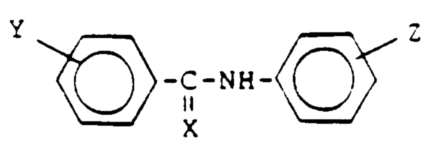

(worin X, Y und Z die obengenannten Bedeutungen aufweisen) umsetzt, und gegebenenfalls danach

eine Reduktion der so erhaltenen Verbindung, falls X = O, und/oder die Umwandlung in ein pharmazeutisch verträgliches Salz ausführt.

**2.** Verfahren nach Anspruch 1, dadurch gekennzeichnet, daß R einen Isobutyl-, Methyl- oder Cyclohexylrest darstellt.

**3.** Verfahren nach Anspruch 1, dadurch gekennzeichnet, daß R' den Methyl- oder n-Propylrest darstellt.

**4.** Verfahren nach Anspruch 1, dadurch gekennzeichnet, daß es sich um 1-Methyl-2-[(2-methylpropoxy)-methyl]-N-benzyl-N-(3,4-methylendioxyd)phenyl-3-piperidinamin oder ein Salz davon handelt.

**5.** Verfahren nach Anspruch 1, dadurch gekennzeichnet, daß die Umsetzung in Anwesenheit einer starken Base wie Natriumamid oder Lithiumamid erfolgt.

**6.** Verfahren nach Anspruch 1, dadurch gekennzeichnet, daß die Umsetzung durch Phasentransfer in Anwesenheit einer starken Base und eines Katalysators wie Benzyltriethylammoniumchlorid erfolgt.

**7.** Verfahren nach Anspruch 1, dadurch gekennzeichnet, daß man die Halogenderivate der Formel I oder II durch Behandlung eines 2-($\alpha$-Hydroxy-$\beta$-alkoxy)ethyl-pyrrolidins oder-piperidins mit einem Halogenierungsmittel erhält.

**8.** Verfahren nach Anspruch 7, dadurch gekennzeichnet, daß die Halogenierung mittels Thionylchlorid erfolgt.